(19) Europäisches Patentamt
European Patent Office
Office européen des brevets

(11) EP 3 977 977 B1

(12) **EUROPEAN PATENT SPECIFICATION**

(45) Date of publication and mention of the grant of the patent:
**12.06.2024 Bulletin 2024/24**

(21) Application number: **20199267.4**

(22) Date of filing: **30.09.2020**

(51) International Patent Classification (IPC):
***A61K 8/46*** *(2006.01)* ***A61Q 5/02*** *(2006.01)*

(52) Cooperative Patent Classification (CPC):
**A61Q 5/02; A61K 8/466**

(54) **CLEANSING COMPOSITION COMPRISING INTERNAL OLEFIN SULFONATE SURFACTANT**

REINIGUNGSMITTEL MIT INNEREM OLEFINSULFONATTENSID

COMPOSITION DE NETTOYAGE COMPRENANT UN AGENT TENSIOACTIF DE SULFONATE D'OLÉFINE INTERNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.04.2022 Bulletin 2022/14**

(73) Proprietor: **Kao Corporation**
**Chuo-ku,**
**Tokyo 103-8210 (JP)**

(72) Inventors:
- **Weitzel, Johanna**
  **64297 Darmstadt (DE)**
- **Pyrozok, Erik**
  **64297 Darmstadt (DE)**
- **Maruyama, Aguri**
  **64297 Darmstadt (DE)**
- **Burgdorf, Kristin**
  **64297 Darmstadt (DE)**

(74) Representative: **Hoffmann Eitle**
**Patent- und Rechtsanwälte PartmbB**
**Arabellastraße 30**
**81925 München (DE)**

(56) References cited:
**WO-A1-2014/112522** **WO-A1-2015/141863**
**WO-A2-2014/046303**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 977 977 B1

## Description

### Field of the invention

The present invention is directed to a cleansing composition and method for cleansing of keratin fibers.

### Background of the invention

Cleansing compositions play an important role in human life, especially in cosmetics. Their primary purpose, of course, is cleansing itself. However, nowadays customers demand products, which possess many facets beyond pure cleansing.

An important feature of cleansing compositions is their ability to produce foam. Good foam properties are commonly associated with purity and wellness. It is well-known that customers prefer fine foams, which have rich and creamy feel and a luxurious appearance. Additionally, the customers expect that the foam lasts for a considerate period to fully enjoy and benefit from the bubbly experience. For a subgroup of customers, beautiful foam sensations positively reflect on their mental wellbeing.

Anionic surfactants, particularly, alkyl sulfates and alkyl polyoxyalkylene sulfates, are excellent in detergency and foaming power, and thus are widely used as cleansing ingredients for domestic or industrial use. An olefin sulfonate, particularly, an internal olefin sulfonate obtained with an internal olefin having a double bond inside an olefin chain, and not at its end, has been reported as one of the anionic surfactants. Such an internal olefin sulfonate is generally obtained by sulfonating an internal olefin through reactions with a gaseous sulfur trioxide-containing gas, followed by neutralization and then hydrolysis of the resulting sulfonic acid.

The internal olefin sulfonate is known to have good biodegradability, but is still insufficient in its basic performance as cleansing agent including foamability and foam quality, compared with general-purpose surfactants such as salts of alkyl sulfates. Thus, further improvement in such basic performance is desired.

EP2897575 addresses this problem and discloses a combination of internal olefin sulfonates with non-ionic or cationic surfactants. In any case, the stability of the foam is of particular concern.

WO 2014/046303 A2 concerns a cleansing composition for skin or hair, which is said to provide a good rinse feel and durability of foam and, in regard to hair, impart finger combability during rinsing as well as softness to hair during rinsing and after towel drying. The composition comprises the following (A) and (B): (A) an internal olefin sulfonate having 12 or more and 24 or less carbon atoms; and (B) a cationic polymer or an amphoteric polymer.

WO 2015/141863 A1 relates to a cosmetic composition containing in an aqueous medium, at least an internal olefin sulfonate composition containing an internal olefin sulfonate having 16 carbon atoms and an internal olefin sulfonate having 18 carbon atoms, and at least one polymeric suspension agent. WO 2014/112522A1 is directed to a method for producing an internal olefin using a primary aliphatic alcohol having from 8 to 24 carbon atoms as a raw material, comprising certain steps (1) - (3). Step (1): A step for dehydrating a primary aliphatic alcohol having from 8 to 24 carbon atoms in the presence of a solid catalyst. Step (2): A step for adjusting the water content of the water-containing olefin obtained by dehydration to from 0.001 to 7 mass%. Step (3): A step for carrying out internal isomerization of the water-containing olefin adjusted to a water content of from 0.001 to 7 mass%, in the presence of a solid catalyst.

### Summary of the invention

Therefore, the first object of the present invention is a cleansing composition for keratin fibers, preferably for human keratin fibers, more preferably for human hair, having a pH in the range of 1 to 7 comprising:

a) one or more amphoteric and/or zwitterionic surfactant(s), and/or their salt(s),

b) one or more non-ionic surfactant(s),

c) one or more internal olefin sulfonate surfactant(s) obtained by sulfonating an internal olefin having 16 carbon atoms and having an average double bond position in the range from 3.9 to 4.5, and/or its salt(s),

wherein the weight ratio of compounds according to group a) to compounds according to group c) is in the range of 2 to 15.

The second object of the present invention is a method for cleansing of keratin fibers, preferably human keratin fibers, more preferably human hair, comprising the steps of:

i) wetting the keratin fibers with water and applying the composition as defined above onto the keratin fibers,

ii) massaging the composition for a time period of 10 s to 10 min,

iii) rinsing-off the composition and optionally drying the keratin fibers.

## Detailed description of the invention

Present inventors have unexpectedly found out that the combination of a specific internal olefin sulfonate surfactant with non-ionic and amphoteric/ zwitterionic surfactants improves foam quality and stability. A fine and creamy foam was observed that lasted for 5 Minutes and more. Improved foamability, volume of foam, foaming speed, and foam dissipation properties were observed. None of the investigated individual or surfactant combinations reached this performance.

### Cleansing composition

The present invention is directed to a cleansing composition for keratin fibers, preferably for human keratin fibers, more preferably for human hair, having a pH in the range of 1 to 7 comprising:

a) one or more amphoteric and/or zwitterionic surfactant(s), and/or their salt(s),

b) one or more non-ionic surfactant(s),

c) one or more internal olefin sulfonate surfactant(s) obtained by sulfonating an internal olefin having 16 carbon atoms and having an average double bond position from 3.9 and 4.5, and/or its salt(s),

wherein the weight ratio of compounds according to group a) to compounds according to group c) is in the range of 2 to 15.

It is preferred from the viewpoint of cosmetic safety and skin compatibility that the pH of the composition is 2 or more, more preferably it is 3 or more, further more preferably it is 4 or more, still further more preferably it is 4.5 or more.

It is preferred from the viewpoint of skin compatibility that the pH of the composition is 6.5 or less, preferably it is 6.0 or less, further more preferably it is 5.5 or less.

For attaining the above-mentioned effects, it is preferred that the pH of the composition is in the range of 2 to 6.5, preferably in the range of 3 to 6.0, more preferably in the range of 4 to 5.5, further more preferably in the range of 4.5 to 5.5.

It is further preferred from the viewpoint of environmental protection and convenience of use that the composition comprises water at 60% by weight or more, preferably at 70% by weight or more, further more preferably at 80% by weight or more, calculated to the total weight of the composition.

It is further preferred that the weight ratio of compounds according to group a) to compounds according to group c) is 10 or less, further preferably it is 8 or less, from the viewpoint of foam stability.

It is further preferred that the weight ratio of compounds according to group a) to compounds according to group c) is 3 or more from the viewpoint of foam stability.

Thus, for attaining the above-mentioned effect, it is preferred that the weight ratio of compounds according to group a) to compounds according to group c) is in the range from 3 to 10, more preferably in the range of 3 to 8.

### Compound(s) according to a)

The composition of the present invention comprises one or more amphoteric and/or zwitterionic surfactant(s), and/or their salt(s).

Suitable amphoteric/zwitterionic surfactants are compounds according to the following general structure(s)

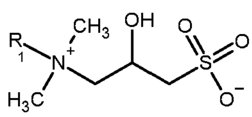

and/or

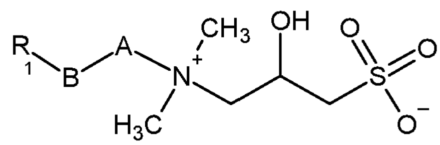

wherein $R_1$ is a straight or branched, saturated or unsaturated, substituted or unsubstituted alkyl chain with a carbon number of $C_{10}$ to $C_{22}$, preferably $R_1$ is a straight alkyl chain with a carbon number of $C_{10}$ to $C_{16}$, A is a straight alkyl chain with a carbon number of $C_1$ to $C_6$ or a branched alkyl chain with a carbon number of $C_3$ to $C_6$, preferably A is a linear alkyl chain with a carbon number of $C_3$, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine, and/or their salt(s).

Further suitable amphoteric/zwitterionic surfactants are alkyl betaine-type surfactants and/or alkyl amidoalkyl betaine-type surfactants, and/or their mixtures.

A preferred compound from alkyl betaines from the viewpoint of foam generation is cocoyl betaine. A preferred compound from alkylamidoalkyl betaines from the viewpoint of foam generation and foam stability is cocamidopropyl betaine. The disclosure also relates to the salts of the compounds.

The most preferred amphoteric/zwitterionic surfactant according to group a) is cocamidopropyl betaine and/or its salt(s) from the viewpoint of foam generation and foam stability.

It is to be noted that the skilled person can combine the surfactants from the classes of above to create suitable surfactant mixtures.

It is preferred from the viewpoint of foam generation that the total concentration of compound(s) according to group a) is 1 % by weight or more, more preferably it is 2% by weight or more, further more preferably it is 3% by weight or more, calculated to the total weight of the composition.

It is preferred from the viewpoint of foam stability that the total concentration of compound(s) according to group a) is 20% by weight or less, more preferably it is 15% by weight or less, further more preferably it is 12% by weight or less, calculated to the total weight of the composition.

For attaining the above-mentioned effects, it is preferred that the total concentration of compound(s) according to group a) is in the range of 1% to 20% by weight, preferably in the range of 2% to 15% by weight, further more preferably in the range of 3% to 12% by weight, calculated to the total weight of the composition.

**Compound(s) according to b)**

The present invention comprises one or more non-ionic surfactant(s) as compound(s) according to group b).

Suitable non-ionic surfactants are of N-alkylpolyhydroxyalkylamide type according to the following general formula:

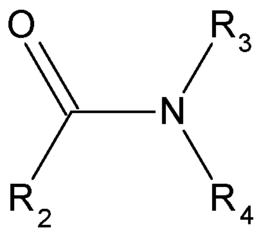

wherein $R_2$ is a linear or branched, saturated or unsaturated alkyl chain with $C_{11}$ to $C_{21}$, $R_3$ is linear or branched alkyl, or linear or branched hydroxyalkyl with $C_1$ to $C_4$, and $R_4$ is a linear or branched polyhydroxyalkyl chain with $C_3$ to $C_{12}$ and 3 to 10 hydroxyl groups.

Such compounds are disclosed in cosmetic compositions in WO96/27366 and their synthesis is disclosed in US1985424, US2016962, US2703798, and WO92/06984.

The preferred N-alkylpolyhydroxyalkylamide type surfactants have the following structure:

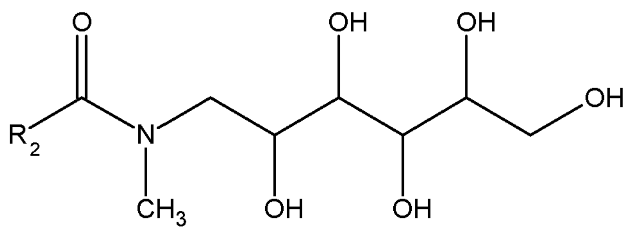

where $R_2$ has the same denotation as above for the general structure of N-alkylpolyhydroxyalkylamide type surfactants. The preferred surfactants as displayed above are known as N-methyl-N-acylglucamides.

The most preferred N-alkylpolyhydroxyalkylamide type surfactants are selected from lauroyl/myristoyl methyl glucamide, coco methyl glucamide, and capryloyl caproyl methyl glucamide.

Further suitable non-ionic surfactants are alkyl glycosides or alkyl polyglycosides according to the general

structure:

$$R_5O(R_6O)_tZ_x$$

Wherein Z denotes a carbohydrate with $C_5$ to $C_6$, $R_5$ is an alkyl group with $C_8$ to $C_{18}$, $R_6$ is methyl, ethyl or propyl, t ranges from 0 to 10, and x ranges from 1 to 5. Suitable compounds according to this structure are $C_9$-$C_{11}$ alkylpolyglycoside, the structures disclosed in EP-A 70 074, and JP 2015-123019A.

The preferred compounds according to the structure of above are decyl glucoside, lauryl glucoside, and coco glucoside, and the most preferred one is coco glucoside.

Suitable examples for non-ionic surfactants are fatty alcohol ethoxylates of the following general structure

$$R_7\ (OCH_2CH_2)_{n1}\ OH$$

wherein $R_7$ is straight or branched, saturated or unsaturated alkyl chain which may be synthetic or natural with a C chain length in the range of 8 to 40, preferably 9 to 30 and more preferably 9 to 24 and n1 is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant.

Further suitable nonionic surfactants are polypropylene glycol ethers of fatty alcohol according to general structure

$$R_7\ (OCH_2\text{-}CH_2\text{-}CH_2)_{n2}\ OH$$

wherein $R_7$ is straight or branched, saturated or unsaturated fatty alcohol which may be synthetic or natural with a C chain length in the range of 8 to 40, preferably 9 to 30 and more preferably 9 to 24 and n2 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

$$R_8\ C(O)\ (OCH_2CH_2)_{n2}\ OH$$

wherein $R_8$ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate,

PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15

Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

$$R_9\,C(O)\,(OCH_2\text{-}CH_2\text{-}CH_2)_{n3}\,OH$$

wherein $R_9$ is straight or branched, saturated or unsaturated alkyl group which may be synthetic or natural with a C chain length in the range of 7 to 39, preferably 9 to 29 and more preferably 9 to 23 and n3 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

$$R_{10}\,(OCH_2\text{-}CH_2\text{-}CH_2)_{n4}\,(OCH_2CH_2)_{n5}\,OH$$

wherein $R_{10}$ is straight or branched, saturated or unsaturated alkyl group which may be synthetic or natural with a C chain length in the range of 7 to 39, preferably 9 to 29 and more preferably 9 to 23 and n4 and n5 may be the same or different and are a number in the range of 1 to 40.

Further suitable nonionic surfactants are ethoxylated vegetable oils. Well-known and commonly used examples are ethoxylated castor oil such as PEG-40 hydrogenated castor oil or and PEG-60 hydrogenated castor oil.

It is preferred from the viewpoint of foam generation that one or more compound(s) according to group b) is selected from alkyl glucoside(s)-type surfactants, alkyl polyglucoside(s)-type surfactants, and/or N-alkylpolyhydroxyalkylamide-type surfactants, and/or their mixtures.

It is preferred from the viewpoint of foam generation that the total concentration of compound(s) according to group b) is 0.5% by weight or more, more preferably 1% by weight or more, further more preferably 2% by weight or more, calculated to the total weight of the composition.

It is preferred from the viewpoint of foam stability that the total concentration of compound(s) according to group b) is 15% by weight or less, more preferably 10% by weight or less, further more preferably 8% by weight or less, calculated to the total weight of the composition.

For attaining the above-mentioned effects, it is preferred that the total concentration of compound(s) according to group b) is in the range of 0.5% to 15% by weight, preferably in the range of 1 % to 10% by weight, more preferably in the range of 2% to 8% by weight, calculated to the total weight of the composition.

**Compound(s) according to c)**

The composition of the present invention comprises one or more internal olefin sulfonate surfactant(s) obtained by sulfonating an internal olefin having 16 carbon atoms and having an average double bond position in the range from 3.9 to 4.5, and/or its salt(s).

It is preferred from the viewpoint of low temperature stability that the average double bond position is 4.0 or higher, more preferably 4.1 or higher.

It is further preferred from the viewpoint of hair washing effect that the average double bond position is 4.4 or less, more preferably 4.3 or less.

For attaining the above-mentioned effect, it is preferred that the average double bond position is in the range of 4.0 to 4.4, more preferably in the range of 4.1 to 4.3.

It is preferred that the average double bond position is determined by gas chromatography coupled with a mass spectrometer detector (GC-MS) of the internal olefin prior to sulfonation.

Calculation of the average double bond position is according to equation 1:

$$\text{Average double bond position in olefin } C_{16} = \sum_{n=1}^{8} \frac{n \cdot C_n}{100}$$

wherein n is an integer representing the position of the double bond present in the raw material olefin with a carbon number of 16 (unit: position), $C_n$ is the concentration by weight of the raw material olefin with a carbon number of 16 having a double bond in n position (unit: % by weight).

It is preferred that the total concentration of compound(s) according to group c) is 0.1% by weight or more, more preferably 0.25% by weight or more, further more preferably 0.5% by weight or more, calculated to the total weight of the composition, form the viewpoint of foam stability.

It is preferred that the total concentration of compound(s) according to group c) is 5% by weight or less, more preferably 4% by weight or less, further more preferably 3% by weight or less, calculated to the total weight of the composition, form the viewpoint of foam stability.

For attaining the above-mentioned effects, it is preferrred that the total concentration of compound(s) according to group c) is in the range of 0.1% to 5% by weight, preferably in the range of 0.25% to 4% by weight, further more preferably in the range of 0.5% to 3% by weight, calculated to the total weight of the composition.

**Method of producing internal olefin sulfonate surfactant according to group c)**

The internal olefin sulfonate composition may be produced by sulfonating a raw material internal olefin having 16 carbon atoms, followed by neutralization, and hydrolysis. More specifically, for example, the composition may be produced in accordance with the methods described in JP2625150, and Tenside Surf. Det. 31 (5) 299 (1994).

As mentioned above, in the present invention, an internal olefin refers to an olefin substantially having a double bond inside the olefin chain. The content of the $\alpha$-olefin in which a double bond is present at a C-1 position in the raw material internal olefin is preferably less than 5.0% by weight. It is further preferred that the $\alpha$-olefin content is 3.0% by weight or less, more preferably it is 2.5% by weight or less, calculated to the total weight of raw material olefin having a carbon number of 16, from the viewpoint of foamability, volume of foam, foaming speed, foam dissipation property, foam quality, and foam durability.

Further, in the raw material olefin having a carbon number of 16, the concentration of the raw material olefin having a double bond in 2 position (n=2) preferably is 10% by weight or more, more preferably 15% by weight or more, further more preferably 20% by weight or more, and preferably 35% by weight or less, more preferably 32% by weight or less still more preferably 24% by weight or less, calculated to the total weight of the raw material olefin having a carbon number of 16. Then, in the raw material olefin having a carbon number of 16, the concentration of the raw material olefin having a double bond in 2 position preferably is in the range from 10% to 35 by weight, more preferably in the range from 15% to 32% by weight, further more preferably in the range from 20% to 24% by weight, calculated to the total weight of the raw material olefin having a carbon number of 16.

In the raw material olefin having a carbon number of 16, the concentration of the raw material olefin having a double bond in 3 position (n=3) preferably is 10% by weight or more, more preferably 14% by weight or more, further more preferably 16% by weight or more, and preferably 30% by weight or less, and more preferably 19% by weight or less, calculated to the total weight of the raw material olefin having a carbon number of 16. Then, in the raw material olefin having a carbon number of 16, the concentration of the raw material olefin having a double bond in 3 position preferably is in the range from 10% to 30% by weight, preferably in the range from 14% to 24% by weight, still more preferably in the range from 16% to 19% by weight, calculated to the total weight of the raw material olefin having a carbon number of 16.

In the raw material olefin having a carbon number of 16, the concentration of the raw material olefin having a double bond in 4 position (n=4) preferably is 10% by weight or more, more preferably 15% by weight or more, further more preferably 17% by weight or more, and preferably 30% by weight or less, more preferably 19% by weight or less, calculated to the total weight of the raw material olefin having a carbon number of 16. Then, in the raw material olefin having a carbon number of 16, the concentration of the raw material olefin having a double bond in position 4 preferably is in the range from 10% to 30% by weight, more preferably in the range from 15% to 25% by weight, further more preferably in the range of 17% to 19% by weight, calculated to the total weight of the raw material olefin having a carbon number of 16.

In the raw material olefin having a carbon number of 16, the concentration of the raw material olefin having a double bond in 5 position (n=5) preferably is 5% by weight or more, more preferably 10% by weight or more, further more preferably 13% by weight or more, and preferably 25% by weight or less, and more preferably 15 by weight or less, calculated to the total weight of the raw material olefin having a carbon number of 16.. Then, in the raw material olefin having a carbon number of 16, the concentration of the raw material olefin having a double bond in 5 position preferably is in the range from 5% to 25% by weight, more preferably 10% to 19% by weight, more preferably 13% to 15% by weight, calculated to the total weight of the raw material olefin having a carbon number of 16.

In the raw material olefin having a carbon number of 16, the concentration of the raw material olefin having a double bond in 6 position (n=6) preferably is 5% by weight or more, more preferably 7% by weight or more, further more preferably 11% by weight or more, and preferably 20% by weight or less, more preferably 13% by weight or less, calculated to the total weight of the raw material olefin having a carbon number of 16. Then, In the raw material olefin having a carbon number of 16, the concentration of the raw material olefin having a double bond in 6 position preferably is in the range from 5% to 20% by weight, more preferably in the range from 7% to 15% by weight, further more preferably in the range from 11 % to 13% by weight, calculated to the total weight of the raw material olefin having a carbon number of 16.

In the raw material olefin having a carbon number of 16, the total concentration of the raw material olefin having

a double bond position i 7-position or 8-position (n=7 or n=8), in total preferably is 5% by weight or more, more preferably 7% by weight or more, further more preferably 12% by weight or more, and preferably 25% by weight or less, and more preferably 22% by weight or less, further more preferably 16% by weight or less, calculated to the total weight of the raw material olefin having a carbon number of 16. Then, in the raw material olefin having a carbon number of 16, the total concentration of the raw material olefin having a double bond in 7-position or 8-position, preferably is in the range of 5% to 25% by weight, more preferably in the range of 7% to 22% by weight, more preferably 12% to 16% by weight, calculated to the total weight of the raw material olefin having a carbon number of 16.

In the raw material olefin having a carbon number of 16, the mass ratio of the concentration of the raw material olefin having a double bond position is 3-5 position (n=3 to n=5) to the concentration of the raw material olefin having a double bond position in 6-8 position (raw material olefin 3-5 position / raw material olefin 6-8 position) is preferably 1.0 or more, more preferably 1.3 or more, further more preferably 1.7 or more, and preferably 4.0 or less, more preferably 3.5 or less, further more preferably 2.2 or less. Then, In the raw material olefin having a carbon number of 16, the mass ratio of the concentration of the raw material olefin having a double bond position is 3-5 position (n=3 to n=5) to the concentration of the raw material olefin having a double bond position in 6-8 position (raw material olefin 3-5 position / raw material olefin 6-8 position) preferably is in the range from 1.0 to 4.0, more preferably in the range of 1.3 to 3.5, and further more preferably in the range of 1.7 to 2.2.

In the synthesis of the internal olefin sulfonate composition, the concentration of the raw material internal olefin in which the double bond is present for each position in the range from C-1 position to C-8 position in the raw material internal olefin may be measured by, for example, by GC-MS. Specifically, components each having different carbon chain lengths and double bond positions are accurately separated by a gas chromatograph analyser, and each component is then analyzed by a mass spectrometer to identify the position of double bond. From the resulting numerical value of GC peak area, the weight fraction of each component is calculated.

**Optional compounds**

The composition of the present invention may further comprise one or more cationic polymer(s), preferably one or more cationic polymer(s) obtained by the reaction of natural carbohydrate-based structure(s) with chemically modified cationic moieties.

Suitable compounds following this definition are guar hydroxyproyltrimonium chloride, starch hydroxypropyltrimonium chloride, cassia hydroxypropyltrimonium chloride, hydroxypropyltrimonium inulin, and Polyquaternium 10, and/or their salt(s), and/or their mixtures.

Suitable concentration ranges for one or more cationic polymer(s), preferably one or more cationic polymer(s) obtained by the reaction of natural carbohydrate base structure(s) with chemically modified cationic moieties preferably are from 0.05% to 1% by weight, more preferably 0.1% to 0.5% by weight, calculated to the total weight of the composition.

The composition may further comprise one or more thickening agent, preferably one or more thickening polymer(s), more preferably one or more non-ionic or anionic thickening polymers, and/or their mixtures.

Suitable non-ionic thickening polymers are, for example, cellulose-based non-ionic polymers such as methylcellulose, ethylcellulose, hydroxypropyl cellulose, hydroxyethylcellulose, or hydroxymethylcellulose.

Further suitable thickening polymers are PEG-based thickening polymers, such as PEG 200 to 4,000.

Suitable anionic thickening polymers are xanthan gum, dehydroxanthan gum, guar gum, and Carbomer-based thickening polymers.

Suitable concentration for thickening agents, preferably for thickening polymers, more preferably for non-ionic and/or anionic thickening polymers, is in the range of 0.1% to 1% by weight, calculated to the total weight of the composition.

The composition of the present invention may further comprise one or more lipophilic compound(s), preferably 1) $C_{12}$ to $C_{22}$ fatty alcohols having a saturated or unsaturated, branched or linear, optionally hydroxylated carbon chain, 2) esters of linear or branched $C_3$ to $C_8$ alcohols with $C_{12}$ to $C_{22}$ fatty acids, 3) natural vegetable oils, 4) petrolatum-based products, 5) silicones, 6) and/or their mixtures.

Suitable fatty alcohols having a linear or branched, saturated or unsaturated carbon chain length of $C_{12}$-$C_{22}$ are, for example, lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures, in particular cetearyl alcohol.

A suitable total concentration of lipophilic compounds is in the range of 0.5% to 10% by weight, preferably 1% to 8% by weight, more preferably 2% to 6% by weight, calculated to the total weight of the composition.

It is preferred from the viewpoint of biodegradability that the composition of the present invention comprises sulfate-based surfactants at 1% by weight or less, more preferably at 0.5% by weight or less, further more preferably at 0.1% by weight or less, still further more preferably it is free of sulfate-based surfactants.

It is further preferred from the viewpoint of customer convenience that the composition of the present invention

is a shampoo composition.

**Compound(s) according to group d)**

It is particularly advantageous from the viewpoint of viscosity that the composition of the present invention comprises one or more non-sulfate-based anionic surfactants as compound(s) according to group d).

Suitable examples are isethionate surfactants, alkylated amino acid surfactants, and sarcosinate surfactants, and/or their salt(s).

The preferred total concentration for these surfactants is in the range of 0.1% to 10% by weight, more preferably in the range of 0.25% to 8% by weight, further more preferably in the range of 0.5% to 5% by weight, calculated to the total weight of the composition.

**Method of cleansing**

The present invention is also directed to a method for cleansing of keratin fibers, preferably human keratin fibers, more preferably human hair, comprising the steps of:

i) wetting the keratin fibers with water and applying the composition as defined above onto the keratin fibers,

ii) massaging the composition for a time period of 10 s to 10 min,

iii) rinsing-off the composition and optionally drying the keratin fibers.

It is preferred from the viewpoint of user convenience that the massaging time is in the range of 30 s to 5 min.

The following examples are to illustrate the invention, but not to limit it.

**EXAMPLES**

**Table 1**

| **Ingredients** | | **Comp. ex.1** | **Comp. ex.2** | **Comp. ex.3** | **Comp. ex.4** | **Comp. ex.5** | **Inv. ex. 1** | **Inv. ex. 2** |
|---|---|---|---|---|---|---|---|---|
| **a)** | **Cocoamidopropyl betaine** | 15.0 | - | - | 10.0 | 7.0 | 10.0 | 7.0 |
| **b)** | **Capryloyl/ caproyl methyl glucamide** | - | 15.0 | - | 5.0 | 3.0 | 4.0 | 5.0 |
| **c)** | **Internal olefin sulfonate** | - | - | 15.0 | - | 5.0 | 1.0 | 1.85 |
| **-** | **Weigh ratio a) to c)** | - | - | - | - | 1.4 | 10 | 3.8 |
| **-** | **NaOH/HCl** | Ad pH 5.0 | | | | | | |
| **-** | **Water** | Ad 100.0 | | | | | | |
| **Effect** | **# at 1 min** | 130 | 227 | 102 | 123 | 290 | 267 | 375 |
| | **# at 3 min** | 114 | 116 | 0 | 79 | 121 | 140 | 299 |
| | **# at 5 min** | 15 | 104 | 0 | 63 | 54 | 125 | 202 |

As illustrated by the examples of table 1, inventive compositions 1 and 2 delivered a foam, which had fine bubbles with prolonged stability. Comparative example 5 delivered a fine foam upon preparation, but the foam quickly disintegrated and lost stability. None of the comparative examples delivered foam with such fine bubbles. Additionally, foam stability was lower for the comparative examples.

**Table 2**

| Ingredients | | Comp. ex.1 | Comp. ex.7 | Comp. ex.3 | Comp. ex.8 | Comp. ex.9 | Inv. ex.3 | Inv. ex.4 |
|---|---|---|---|---|---|---|---|---|
| a) | **Cocoamidopropyl betaine** | 15.0 | - | - | 10.0 | 7.0 | 10.0 | 7.0 |
| b) | **Coco-glycoside** | - | 15.0 | - | 5.0 | 3.0 | 4.0 | 5.0 |
| c) | **Internal olefin sulfonate** | - | - | 15.0 | - | 5.0 | 1.0 | 1.85 |
| - | **Weigh ratio a) to c)** | - | - | - | - | 1.4 | 10 | 3.8 |
| - | **NaOH/HCl** | Ad pH 5.0 | | | | | | |
| - | **Water** | Ad 100.0 | | | | | | |
| **Effect** | **# at 1 min** | 130 | 180 | 102 | 114 | 114 | 177 | 162 |
| | **# at 3 min** | 114 | 89 | 0 | 54 | 70 | 91 | 95 |
| | **# at 5 min** | 15 | 49 | 0 | 21 | 49 | 62 | 92 |

As illustrated by the examples of table 2, inventive compositions 3 and 4 delivered a foam, which had fine bubbles with prolonged stability. None of the comparative examples delivered foam with such fine bubbles. Additionally, foam stability was lower for the comparative examples.

**Table 3**

| Ingredients | | Inv ex.5 | Inv ex.6 | Inv ex.7 | Inv ex.8 | Inv ex.9 | Inv ex.10 |
|---|---|---|---|---|---|---|---|
| a) | **Cocoamidopropyl betaine** | 7.0 | 7.0 | 7.0 | 7.0 | 7.0 | 5.0 |
| b) | **Coco-glycoside** | 5.0 | 5.0 | 5.0 | 5.0 | 3.0 | 5.0 |
| c) | **Internal olefin sulfonate** | 2.0 | 2.0 | 2.0 | 2.0 | 2.0 | 2.0 |
| d) | **Sodium cocoyl isethionate** | - | 1.0 | - | - | | - |
| | **Sodium methyl cocoyl taurate** | - | - | 1.0 | - | | - |
| | **Sodium cocoyl glycinate** | - | - | - | 1.0 | | - |
| | **Sodium lauroyl cocoyl sarcosinate** | - | - | - | - | 2.0 | 2.0 |
| - | **Weigh ratio a) to c)** | 3.5 | 3.5 | 3.5 | 3.5 | 3.5 | 2.5 |
| - | **NaOH/HCl** | Ad pH 5.0 | | | | | |
| - | **Water** | Ad 100.0 | | | | | |
| **Effect** | **Viscosity [mPas]** | 5,000 | 20,200 | 13,900 | 17,400 | 31,480 | 15,000 |

As illustrated by the examples of table 3, the addition of a surfactant according to group d) additionally improved viscosity of the compositions.

## Methods

### Foam microscopy

The compositions from above were diluted with water in a weight ratio of 1:10. The dilution was foamed up in a glass beaker beaker by stirring it with a magent stirrer at 300 rpm for 5 min.

The resulting foam was applied onto glass slides. The samples were investigated with an Olympus light microscope at 10x magnification. Images were taken from the middle of the glass slides.

Image analysis was conducted with Imagej software. The contrast was adjusted by hand and particle numbers were analyzed by automatic mode.

### Viscosity

50 g of the compositions from above were placed in a tall, thin, beaker and used for viscosity measurements in the Brookfield viscometer and spindle #5 at 25°C at a shear rate of 10 rpm.

Measurements were carried out for 1 min and the measurement value for dynamic viscosity was taken after 1 min of shearing. An average of 3 measurements was reported.

## Claims

1. A cleansing composition for keratin fibers, preferably for human keratin fibers, more preferably for human hair, having a pH in the range of 1 to 7 comprising:

    a) one or more amphoteric and/or zwitterionic surfactant(s), and/or their salt(s),
    b) one or more non-ionic surfactant(s),
    c) one or more internal olefin sulfonate surfactant(s) obtained by sulfonating an internal olefin having 16 carbon atoms and having an average double bond position in the range from 3.9 to 4.5, and/or its salt(s),

    wherein the weight ratio of compounds according to group a) to compounds according to group c) is in the range of 2 to 15.

2. The composition according to claim 1 **characterized in that** the pH of the composition is in the range of 2 to 6.5, preferably in the range of 3 to 6.0, more preferably in the range of 4 to 5.5, further more preferably in the range of 4.5 to 5.5.

3. The composition according to claims 1 and/or 2 **characterized in that** it comprises water at 60% by weight or more, preferably at 70% by weight or more, more preferably at 80% by weight or more, calculated to the total weight of the composition.

4. The composition according to any of the preceding claims **characterized in that** it one or more compound(s) according to group a) is selected from alkyl betaine-type surfactants and/or alkyl amidoalkyl betaine-type surfactants, and/or their mixtures.

5. The composition according to any of the preceding claims **characterized in that** the total concentration of compound(s) according to group a) is in the range of 1% to 20% by weight, preferably in the range of 2% to 15% by weight, more preferably in the range of 3% to 12% by weight, calculated to the total weight of the composition.

6. The composition according to any of the preceding claims **characterized in that** one or more compound(s) according to group b) is/are selected from alkyl glucoside(s)-type surfactants, alkyl polyglucoside(s)-type surfactants, and/or N-alkylpolyhydroxyalkylamide-type surfactants, and/or their mixtures.

7. The composition according to any of the preceding claims **characterized in that** the total concentration of compound(s) according to group b) is in the range of 0.5% to 15% by weight, preferably in the range of 1% to 10% by weight, more preferably in the range of 2% to 8% by weight, calculated to the total weight of the composition.

8. The composition according to any of the preceding claims **characterized in that** the total concentration of compound(s) according to group c) is in the range of 0.1% to 5% by weight, preferably in the range of 0.25% to 4% by weight, more preferably in the range of 0.5% to 3% by weight, calculated to the total weight of the composition.

9. The composition according to any of the preceding claims **characterized in that** it comprises one or more cationic polymer(s), preferably one or more cationic polymer(s) obtained by the reaction of natural carbohydrate based structure(s) with chemically modified cationic moieties.

10. The composition according to any of the preceding claims **characterized in that** it comprises one or more thickening agent, preferably one or more thickening polymer(s), more preferably one or more non-ionic or anionic thickening polymers, and/or their mixtures.

11. The composition according to any of the preceding claims **characterized in that** it comprises one or more lipophilic compound(s), preferably 1) C12 to C22 fatty alcohols having a saturated or unsaturated, branched or linear, optionally hydroxylated carbon chain, 2) esters of linear or branched C3 to C8 alcohols with C12 to C22 fatty acids, 3) natural vegetable oils, 4) petrolatum-based products, 5) silicones, 6) and/or their mixtures.

12. The composition according to any of the preceding claims **characterized in that** the total concentration of sulfate-based surfactants is 1% by weight or less, preferably 0.5% by weight or less, more preferably 0.1% by weight or less, further more preferably it is free of sulfate-based surfactants.

**13.** The composition according to any of the preceding claims **characterized in that** it is a shampoo composition.

**14.** The composition according to any of the preceding claims **characterized in that** it comprises one or more non-sulfate-based anionic surfactants as compound(s) according to group d).

**15.** A method for cleansing of keratin fibers, preferably human keratin fibers, more preferably human hair, comprising the steps of:

i) wetting the keratin fibers with water and applying the composition as defined in the claims 1 to 14 onto keratin fibers,
ii) massaging the composition for a time period of 10 s to 10 min,
iii) rinsing-off the composition and optionally drying the keratin fibers.

## Patentansprüche

**1.** Reinigungszusammensetzung für Keratinfasern, bevorzugt für menschliche Keratinfasern, mehr bevorzugt für menschliches Haar, die einen pH-Wert im Bereich von 1 bis 7 aufweist, umfassend:

a) ein oder mehrere amphotere(s) und/oder zwitterionische(s) Tensid(e) und/oder deren Salz(e),
b) ein oder mehrere nicht-ionische(s) Tensid(e),
c) ein oder mehrere interne(s) Olefinsulfonat-Tensid(e), erhalten durch Sulfonieren eines internen Olefins mit 16 Kohlenstoffatomen und einer durchschnittlichen Doppelbindungsposition im Bereich von 3,9 bis 4,5 und/oder ihr(e) Salz(e),

wobei das Gewichtsverhältnis von Verbindungen gemäß Gruppe a) zu Verbindungen gemäß Gruppe c) im Bereich von 2 bis 15 liegt.

**2.** Zusammensetzung gemäß Anspruch 1, **dadurch gekennzeichnet, dass** der pH-Wert der Zusammensetzung im Bereich von 2 bis 6,5, bevorzugt im Bereich von 3 bis 6,0, mehr bevorzugt im Bereich von 4 bis 5,5, noch mehr bevorzugt im Bereich von 4,5 bis 5,5, liegt.

**3.** Zusammensetzung gemäß Anspruch 1 und/oder 2, **dadurch gekennzeichnet, dass** sie, berechnet auf das Gesamtgewicht der Zusammensetzung, Wasser zu 60 Gew.-% oder mehr, bevorzugt zu 70 Gew.-% oder mehr, mehr bevorzugt zu 80 Gew.-% oder mehr, enthält.

**4.** Zusammensetzung gemäß einem der vorhergehenden Ansprüche, **dadurch gekennzeichnet, dass** sie eine oder mehrere Verbindung(en) gemäß Gruppe a), ausgewählt aus Tensiden vom Alkylbetain-Typ und/oder Tensiden vom Alkylamidoalkylbetain-Typ und/oder deren Mischungen, enthält.

**5.** Zusammensetzung gemäß einem der vorhergehenden Ansprüche, **dadurch gekennzeichnet, dass** die Gesamtkonzentration der Verbindung(en) gemäß Gruppe a) im Bereich von 1 bis 20 Gew.-%, bevorzugt im Bereich von 2 bis 15 Gew.-%, mehr bevorzugt im Bereich von 3 bis 12 Gew.-%, berechnet auf das Gesamtgewicht der Zusammensetzung, liegt.

**6.** Zusammensetzung gemäß einem der vorhergehenden Ansprüche, **dadurch gekennzeichnet, dass** eine oder mehrere Verbindung(en) gemäß Gruppe b) aus Tensiden vom Alkylglucosid-Typ, Tensiden vom Alkylpolyglucosid-Typ und/oder Tensiden vom N-Alkylpolyhydroxyalkylamid-Typ und/oder deren Mischungen ausgewählt ist/sind.

**7.** Zusammensetzung gemäß einem der vorhergehenden Ansprüche, **dadurch gekennzeichnet, dass** die Gesamtkonzentration der Verbindung(en) gemäß Gruppe b) im Bereich von 0,5 bis 15 Gew.-%, bevorzugt im Bereich von 1 bis 10 Gew.-%, mehr bevorzugt im Bereich von 2 bis 8 Gew.-%, berechnet auf das Gesamtgewicht der Zusammensetzung, liegt.

**8.** Zusammensetzung gemäß einem der vorhergehenden Ansprüche, **dadurch gekennzeichnet, dass** die Gesamtkonzentration der Verbindung(en) gemäß Gruppe c) im Bereich von 0,1 bis 5 Gew.-%, bevorzugt im Bereich von 0,25 bis 4 Gew.-%, mehr bevorzugt im Bereich von 0,5 bis 3 Gew.-%, berechnet auf das Gesamtgewicht der Zusammensetzung, liegt.

**9.** Zusammensetzung gemäß einem der vorhergehenden Ansprüche, **dadurch gekennzeichnet, dass** sie ein oder mehrere kationische(s) Polymer(e), bevorzugt ein oder mehrere kationische(s) Polymer(e), erhalten durch die Reaktion von Strukturen auf natürlicher Kohlenhydratbasis mit chemisch modifizierten kationischen Gruppen, umfasst.

**10.** Zusammensetzung gemäß einem der vorhergehenden Ansprüche, **dadurch gekennzeichnet, dass** sie ein oder mehrere Verdickungsmittel, bevorzugt ein oder mehrere verdickende(s) Polymer(e), mehr bevorzugt ein oder mehrere nichtionische oder anionische verdickende Polymere, und/oder deren Mischungen umfasst.

**11.** Zusammensetzung gemäß einem der vorhergehenden Ansprüche, **dadurch gekennzeichnet, dass** sie eine oder mehrere lipophile Verbindung(en), bevorzugt 1) C12- bis C22-Fettalkohole mit einer gesättigten oder ungesättigten, verzweigten oder linearen, optional hydroxylierten Kohlenstoffkette, 2) Ester von linearen oder verzweigten C3- bis C8-Alkoholen mit C12- bis C22-Fettsäuren, 3) natürliche pflanzliche Öle, 4) Produkte auf Petrolatum-Basis, 5) Silikone, 6) und/oder deren Mischungen umfasst.

**12.** Zusammensetzung gemäß einem der vorhergehenden Ansprüche, **dadurch gekennzeichnet, dass** die Gesamtkonzentration an sulfatbasierten Tensiden 1 Gew.-% oder weniger, bevorzugt 0,5 Gew.-% oder weniger, mehr bevorzugt 0,1 Gew.-% oder weniger, beträgt und sie noch mehr bevorzugt frei von sulfatbasierten Tensiden ist.

**13.** Zusammensetzung gemäß einem der vorhergehenden Ansprüche, **dadurch gekennzeichnet, dass** sie eine Shampoo-Zusammensetzung ist.

**14.** Zusammensetzung gemäß einem der vorhergehenden Ansprüche, **dadurch gekennzeichnet, dass** sie ein oder mehrere anionische nicht-sulfatbasierte Tenside als Verbindung(en) gemäß Gruppe d) enthält.

**15.** Verfahren zur Reinigung von Keratinfasern, bevorzugt menschlichen Keratinfasern, mehr bevorzugt menschlichem Haar, umfassend die folgenden Schritte:

i) das Befeuchten der Keratinfasern mit Wasser und das Auftragen der Zusammensetzung, wie in den Ansprüchen 1 bis 14 definiert, auf die Keratinfasern,
ii) das Einmassieren der Zusammensetzung über einen Zeitraum von 10 s bis 10 min,
iii) das Abspülen der Zusammensetzung und optional das Trocknen der Keratinfasern.

**Revendications**

**1.** Composition de nettoyage pour fibres kératiniques, de préférence pour fibres kératiniques humaines, de manière encore préférée pour cheveux humains, présentant un pH dans la plage de 1 à 7 comprenant :

a) un ou plusieurs tensioactif(s) amphotère(s) et/ou zwittérionique(s), et/ou ses ou leur(s) sel(s),
b) un ou plusieurs tensioactif(s) non-ionique(s),
c) un ou plusieurs tensioactif(s) au sulfonate d'oléfine interne obtenu(s) par la sulfonation d'une oléfine interne présentant 16 atomes de carbone et présentant une position de liaison double moyenne dans la plage de 3,9 à 4,5, et/ou son ou ses sel(s), dans lequel le rapport en poids des composés selon le groupe a) sur les composés selon le groupe c) est dans la plage de 2 à 15.

**2.** Composition selon la revendication 1 **caractérisée en ce que** le pH de la composition est dans la plage de 2 à 6,5, de préférence dans la plage de 3 à 6,0, de manière encore préférée dans la plage de 4 à 5,5, de manière encore davantage préférée dans la plage de 4,5 à 5,5.

**3.** Composition selon la revendication 1 et/ou la revendication 2 **caractérisée en ce qu'**elle comprend de l'eau à hauteur de 60 % en poids ou plus, de préférence à hauteur de 70 % en poids ou plus, de manière encore préférée à hauteur de 80 % en poids ou plus, calculée par rapport au poids total de la composition.

**4.** Composition selon l'une quelconque des revendications précédentes **caractérisée en ce qu'**un ou plusieurs composé(s) selon le groupe a) est/sont sélectionné(s) parmi les tensioactifs de type alkylbétaïne et/ou les tensioactifs de type alkylamidoalkylbétaïne, et/ou leurs mélanges.

**5.** Composition selon l'une quelconque des revendications précédentes **caractérisée en ce que** la concentration

totale en composé(s) selon le groupe a) est dans la plage de 1 % à 20 % en poids, de préférence dans la plage de 2 % à 15 % en poids, de manière encore préférée dans la plage de 3 % à 12 % en poids, calculée par rapport au poids total de la composition.

6. Composition selon l'une quelconque des revendications précédentes **caractérisée en ce qu'**un ou plusieurs composé(s) selon le groupe b) est/sont sélectionné(s) parmi les tensioactifs de type alkylglucoside(s), les tensioactifs de type alkylpolyglucoside(s), et/ou les tensioactifs de type N-alkylpolyhydroxyalkylamide, et/ou leurs mélanges.

7. Composition selon l'une quelconque des revendications précédentes **caractérisée en ce que** la concentration totale en composé(s) selon le groupe b) est dans la plage de 0,5 % à 15 % en poids, de préférence dans la plage de 1 % à 10 % en poids, de manière encore préférée dans la plage de 2 % à 8 % en poids, calculée par rapport au poids total de la composition.

8. Composition selon l'une quelconque des revendications précédentes **caractérisée en ce que** la concentration totale en composé(s) selon le groupe c) est dans la plage de 0,1 % à 5 % en poids, de préférence dans la plage de 0,25 % à 4 % en poids, de manière encore préférée dans la plage de 0,5 % à 3 % en poids, calculée par rapport au poids total de la composition.

9. Composition selon l'une quelconque des revendications précédentes **caractérisée en ce qu'**elle comprend un ou plusieurs polymère(s) cationique(s), de préférence un ou plusieurs polymère(s) cationique(s) obtenus par la réaction de structure(s) à base de glucides naturels avec des fractions cationiques chimiquement modifiées.

10. Composition selon l'une quelconque des revendications précédentes **caractérisée en ce qu'**elle comprend un ou plusieurs agents épaississants, de préférence un ou plusieurs polymère(s) épaississant(s), de manière encore préférée un ou plusieurs polymères épaississants non ioniques ou anioniques, et/ou leurs mélanges.

11. Composition selon l'une quelconque des revendications précédentes **caractérisée en ce qu'**elle comprend un ou plusieurs composé(s) lipophile(s), de préférence 1) des alcools gras en C12 à C22 présentant une chaîne carbonée saturée ou insaturée, ramifiée ou linéaire, facultativement hydroxylée, 2) des esters d'alcools linéaires ou ramifiés en C3 à C8 avec des acides gras en C12 à C22, 3) des huiles végétales naturelles, 4) des produits à base de pétrolatum, 5) des silicones, 6) et/ou leurs mélanges.

12. Composition selon l'une quelconque des revendications précédentes **caractérisée en ce que** la concentration totale en tensioactifs à base de sulfate est de 1 % en poids ou moins, de préférence de 0,5 % en poids ou moins, de manière encore préférée de 0,1 % en poids ou moins, de manière encore davantage préférée elle est exempte de tensioactifs à base de sulfate.

13. Composition selon l'une quelconque des revendications précédentes **caractérisée en ce qu'**elle est une composition de shampooing.

14. Composition selon l'une quelconque des revendications précédentes **caractérisée en ce qu'**elle comprend un ou plusieurs tensioactifs anioniques non à base de sulfate en tant que composé(s) selon le groupe d).

15. Procédé pour le nettoyage de fibres kératiniques, de préférence de fibres kératiniques humaines, de manière encore préférée de cheveux humains, comprenant les étapes suivantes :

    i) le mouillage des fibres kératiniques avec de l'eau et l'application de la composition telle que définie selon les revendications 1 à 14 sur les fibres kératiniques,
    ii) la réalisation d'un massage avec la composition pendant une durée de 10 s à 10 min,
    iii) le rinçage de la composition et facultativement le séchage des fibres kératiniques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2897575 A
- WO 2014046303 A2
- WO 2015141863 A1
- WO 2014112522 A1
- WO 9627366 A
- US 1985424 A
- US 2016962 A
- US 2703798 A
- WO 9206984 A
- EP 70074 A
- JP 2015123019 A
- JP 2625150 B


### Non-patent literature cited in the description


- *Tenside Surf. Det.,* 1994, vol. 31 (5), 299